Europäisches Patentamt

European Patent Office

Office européen des brevets

(19)

(11) Veröffentlichungsnummer : **0 163 891**
**B1**

(12) **EUROPÄISCHE PATENTSCHRIFT**

(45) Veröffentlichungstag der Patentschrift :
02.03.88

(21) Anmeldenummer : 85104842.1

(22) Anmeldetag : 22.04.85

(51) Int. Cl.⁴ : **A 41 G 1/00**, D 04 H 1/00,
A 61 F 13/20

(54) **Verfahren zum Herstellen des Duftträgers einer Kunstblume und nach dem Verfahren hergestellter Duftträger.**

(30) Priorität : 30.04.84 DE 3416007

(43) Veröffentlichungstag der Anmeldung :
11.12.85 Patentblatt 85/50

(45) Bekanntmachung des Hinweises auf die Patenterteilung : 02.03.88 Patentblatt 88/09

(84) Benannte Vertragsstaaten :
AT BE CH DE FR GB IT LI LU NL SE

(56) Entgegenhaltungen :
EP-A- 0 093 251
DE-U- 8 212 730
FR-A- 596 282
FR-A- 636 056
US-A- 1 644 482
US-A- 3 861 991
US-A- 4 081 884

(73) Patentinhaber : **Henkel Kommanditgesellschaft auf
Aktien**
**Postfach 1100 Henkelstrasse 67**
**D-4000 Düsseldorf-Holthausen (DE)**

(72) Erfinder : **Siegers, Hans-Peter**
**Schwalmweg 18**
**D-5144 Wegberg (DE)**
Erfinder : **Macke, Wilfried**
**Beethovenstrasse 16**
**D-4154 Tönisvorst 2 (DE)**

EP 0 163 891 B1

Jouve, 18, rue St-Denis, 75001 Paris, France

## Beschreibung

Die Erfindung betrifft ein Verfahren zum Herstellen des Duftträgers einer duftenden Kunstblume bestehend aus einem saugfähigen Material, wie Vliesstoff, mit den Duftstoff abdampfender Oberfläche. Sie betrifft ferner einen nach dem Verfahren hergestellten Duftträger und dessen Verpackung.

Eine Kunstblume mit einem Duftstoffreservoir wird in dem DE-GM 82 12 730.1 beschrieben. Die Kunstblume soll als Einzelstück oder innerhalb eines Kunstblumenstraußes den Geruch natürlicher Blumen vortäuschen und der Umgebung einen entsprechend angenehmen Duft verleihen. Da Kunstblumen bei fast naturgetreuer Nachgestaltung lange Zeit aufgehoben zu werden pflegen, besteht das Bestreben, dem relativ schnellen Verblassen des Duftes entgegenzuwirken. In dem Gebrauchsmuster wird daher vorgeschlagen, einen Duftdocht aus einem mit dem jeweiligen Duftstoff beaufschlagten gut saugfähigen Material auf einem im Kunstblumen- Kelch angeordneten Untersatz austauschbar zu befestigen. Bei Nachlassen des Duftens kann jeweils ein neuer Duftdocht, d. h. ein neues Duftstoffreservoir, in die Blume eingesetzt werden.

Der bekannte Duftdocht kann aus einem weichen, saugfähigen Vliesstoff bestehen und eine der Blume entsprechende Farbe besitzen. Das Einsetzen des Duftdochtes in die jeweilige Kunstblume wird erleichtert, wenn im Kelch als Untersatz eine Scheibe mit senkrecht nach außen gerichtetem Dorn zum Aufspießen des Dochts vorhanden ist. Schließlich soll es nach dem Gebrauchsmuster vorteilhaft sein, wenn das dem Untersatz zuzuwendende Ende des Duftdochtes in einem flachen Behälter aus Kunststoff fixiert wird, dessen Boden bei Anwendung zwecks Klemmens der Befestigung des Duftdochts mit dem Dorn zu durchstechen ist.

Probleme ergeben sich unter anderem bei dem Herstellen, Befüllen und Verpacken des bekannten Duftdochts aus der Tatsache, daß zugleich eine hohe Saugfähigkeit verlangt wird, ohne daß die Oberfläche des Duftträgers naß wirkt, und daß eine hohe axiale Stabilität sowie ein fester Sitz bei einfachster Montage verlangt werden. Die Saugfähigkeit muß hoch sein, damit der Duftträger bei möglichst kleinem Volumen für eine hohe Betriebsdauer eine möglichst große Duftstoffmenge aufnehmen kann. Eine hohe axiale Stabilität ist notwendig, damit beim Aufstecken und Abnehmen des Duftträgers dessen Form erhalten bleibt.

Beispielsweise Vliesstoffe aus Zellwollfasern besitzen eine hohe Saugfähigkeit, die Stabilität dieses Materials läßt aber zu wünschen übrig. Zum Erhöhen der Formstabilität dieser Fasern können im vorliegenden Zusammenhang auch Bindemittel oder eine Heißverfestigung nach Beimischung von Kunststoffasern nicht herangezogen werden, weil dadurch — abgesehen von den höheren Kosten — die Saugfähigkeit und damit die Lebensdauer vermindert würde.

Auch die nach dem aus der US-PS 4 081 884 bekannten Verfahren aus Vliesstoff durch Verpressen und Erhitzen der radialen Seitenflächen hergestellten Faserrohlinge weisen nicht die für Duftträger notwendige axiale Stabilität auf.

Der Erfindung liegt die Aufgabe zugrunde, ein Verfahren zum Herstellen eines Duftträgers zu schaffen, durch das eine maximale Saugfähigkeit und zugleich eine für die Handhabung ausreichende axiale Stabilität erreicht werden. Die erfindungsgemäße Lösung besteht darin, daß ein aus Zellwollfasern bestehender Vliesstoff durch Nadeln mechanisch verfestigt wird und daß der verfestigte Faserrohling in einer an allen Formflächen beheizten Presse komprimiert wird. Der erfindungsgemäße Duftträger ist gekennzeichnet durch einen Zellwollfaserkörper mit einer Dichte von weniger als 0,6 g/cm$^3$ und mindestens 0,4 g/cm$^3$ Duftstoff-Aufnahmekapazität. Verbesserungen und weitere Ausgestaltungen der Erfindung werden in den Unteransprüchen beschrieben.

Zum Herstellen des erfindungsgemäßen Duftträgers wird als Rohstoff vorzugsweise Zellwolle mit einem Titer von etwa 3,6 dtex und einer Stapellänge von etwa 30 mm verwendet. Die Fasern können entsprechend der Blumenfarbe mit öl- und wasserfester Farbe eingefärbt werden. Bei entsprechend großen Fasermengen kann auch spinndüsengefärbte Zellwolle eingesetzt werden.

Das Verarbeiten zu einem Vliesstoff mit z. B. 600 g/m$^2$ Flächengewicht, wird vorzugsweise auf einer Wirrvliesstraße ausgeführt. Hier können die Fasern zum Aufhellen der Farben auch — gegebenenfalls zweckmäßig in einem festen Verhältnis — mit weißen Fasern gemischt werden. In einem oder mehreren Öffnungsaggregaten werden die Fasern geöffnet und auf einer pneumatisch ablegenden Wirrvlieskarde zu einem Vlies gelegt. Dieses Vlies wird mit einer Nadelmaschine intensiv mechanisch verfestigt.

Durch das Nadeln wird vor allem die verlangte Stabilität beim Aufstecken und Abnehmen des Duftträgers erreicht. Bindemittel und/oder eine Heißverfestigung (nach Beimischung von Kunststoffasern) sind daher zum mechanischen Stabilisieren nicht erforderlich.

Nach dem Nadeln wird der Vliesstoff zweckmäßig in Stückgrößen von 30 × 40 mm geschnitten und einer Backenpresse zugeführt. Der Vliesstoffabschnitt faltet sich in der Backenpresse während des Preßvorgangs beispielsweise W-förmig zu einem radial verpreßten runden Faserrohling. Die Backenpresse kann zum Reduzieren der Faser/Metall-Reibung beheizt werden.

Im Anschluß an die Backenpresse gelangt der Faserrohling in eine geheizte Preßtrommel. Er kann beispielsweise mit Hilfe einer Schubstange aus der Backenpresse in die Preßtrommel eingeschoben und dort axial komprimiert werden. Dabei wird der Faserrohling in seiner Form der Kontur von Preßhülse und Preßstößel angepaßt.

Eine saubere beständige Form des Duftstoffträgers wird nur erreicht, wenn alle Oberflächen — auch der kopfseitige Verschluß und der Preßstößel — beheizt werden. Nach dem Zurückfahren von Kopfverschluß und Preßstößel der Preßtrommel wird der Duftstoffträger zweckmäßig noch für eine gewisse Verweildauer, z. B. etwa 8-15 Sekunden lang, in der Formhülse belassen.

Die Vorbereitung des Faserrohlings und das anschließende Pressen werden so geführt, daß sich eine Dichte des Duftstoffträgers von weniger als 0,6 g/cm³ ergibt, so daß mindestens 0,4 g/cm³ Duftstoff aufgenommen werden kann, ohne daß die Trägeroberfläche naß wirkt. Dieses Ziel wird zugleich mit der erforderlichen mechanischen Stabilität erreicht, wenn das Vlies in der beschriebenen Weise zunächst geöffnet, gelegt und genadelt, vorgepreßt und schließlich heißgepreßt wird.

Da naturgemäß ein so hergestellter Faserpreßling bzw. Duftträger keine hohe Maßgenauigkeit haben kann, eine solche aber für das maschinelle Handhaben und reproduzierbare Befestigen in der Kunstblume notwendig ist, kann es vorteilhaft sein, den Duftträger in einen Kunststoffnapf einzusetzen, dessen Napfdurchmesser im Hinblick auf eine klemmende Halterung des Duftträgers bemessen sein soll. Im Napfboden sollen sich beispielsweise Klemmlippen befinden, die einen aus der Kunststoffblume, insbesondere in deren Kelch, vorspringenden Draht mit definierter Haltekraft nach dem Aufstechen und Schließen umfassen sollen. Die Form des Napfes kann ähnlich den jeweiligen inneren Blütenblättern oder Blütenstempeln gewählt werden. Der verwendete Kunststoff muß natürlich ebenso wie die Fasern gegenüber dem Duftstoff selbst resistent also in der Regel u. a. wasser- und ölfest sein.

Der Duftträger muß vor seinem Gebrauch diffusionsdicht verpackt werden. Gemäß weiterer Erfindung wird die vorgesehene Verpackung sowohl zum Beduften des Duftträgers als auch als Hilfsmittel zum Aufsetzen des bedufteten Duftträgers auf den vorgesehenen Platz in der jeweiligen Kunstblume verwendet. Beispielsweise wird der Duftträger mit dem Kopfende nach vorn in einen flüssigen Duftstoff enthaltenden Kunststoffbecher eingelegt. Der Duftträger saugt den Duftstoff — meist sofort — auf. Nach dem Einlegen des Duftträgers wird der Becher mit einer diffusionsdichten Folie, z. B. einer Aluminiumverbundfolie, versiegelt.

Beim Einsetzen des Duftträgers in eine Kunstblume kann die Verpackung als Fingerschutz dienen, um zu verhindern, daß die Finger mit Duftstoff benetzt werden. Die Verpackung kann dazu als Peel-off-Packung oder als Durchdrückpackung ausgebildet werden. Im einen Fall wird die Abdeckfolie zunächst abgezogen, dann wird der Becher mit den Fingern geklemmt in den Blütenkelch eingeführt und in einer dort vorgesehenen Klemmvorrichtung oder dergleichen verankert. Im anderen Fall wird die Abdeckfolie des Bechers mit dem Haltedraht oder dergleichen der Kunstblume durchstoßen und der Duftträger soweit eingeschoben, bis der Klemm-Mechanismus der Blume wirkt. Daraufhin wird die Verpackung unter Einreißen der Folie zurückgezogen, so daß der Duftträger in der Blume zurückbleibt.

Anhand der schematischen Darstellung von Ausführungsbeispielen werden Einzelheiten der Erfindung erläutert. Es zeigen :

Fig. 1 das Pressen eines Vliesstücks in einer bis 3 Backenpresse ;

Fig. 4 das Pressen des nach Fig. 1 bis 3 vor- und 5 gepreßten Faserrohlings in einer geheizten Preßtrommel ;

Fig. 6 Beispiele von Außenformen des gepreßten und 7 Duftträgers ;

Fig. 8 ein in einem Blütenkelch befestigter Duftträger ;

Fig. 9 ein Duftträger mit angeklemmtem Napf ;

Fig. 10 ein Napf mit Einführtrichter im Querschnitt und in der Ansicht ;

Fig. 11 ein diffusionsdicht verpackter Duftträger im Querschnitt ;

Fig. 12 eine Peel-off-Packung entsprechend Fig. 11 und 13 in der Handhabung beim Aufsetzen auf eine Klemmvorrichtung in einer Kunstblume ; und

Fig. 14 eine Durchdrückpackung entsprechend Fig. bis 16 11 in der Handhabung beim Befestigen an der Klemmvorrichtung einer Kunstblume.

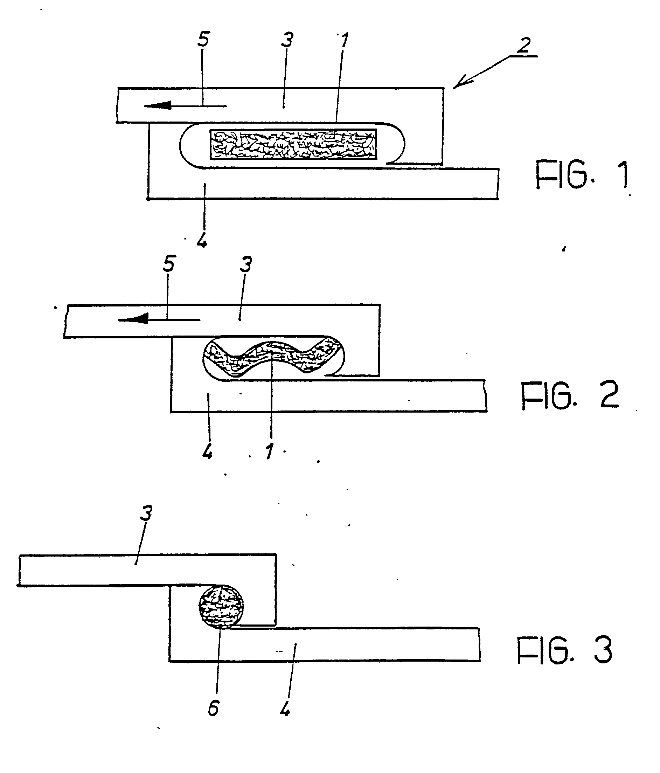

Nach Fig. 1 bis 3 wird ein aus Zellwollfasern bestehender, von einer Wirrvlieskarde kommender Vliesstoffabschnitt 1 mit Hilfe einer Backenpresse 2 durch Gegeneinanderbewegung der Preßbacken 3 und 4 in Pfeilrichtung 5 zunächst W-förmig gestaucht und schließlich zu einem runden Faserrohling 6 radial verpreßt.

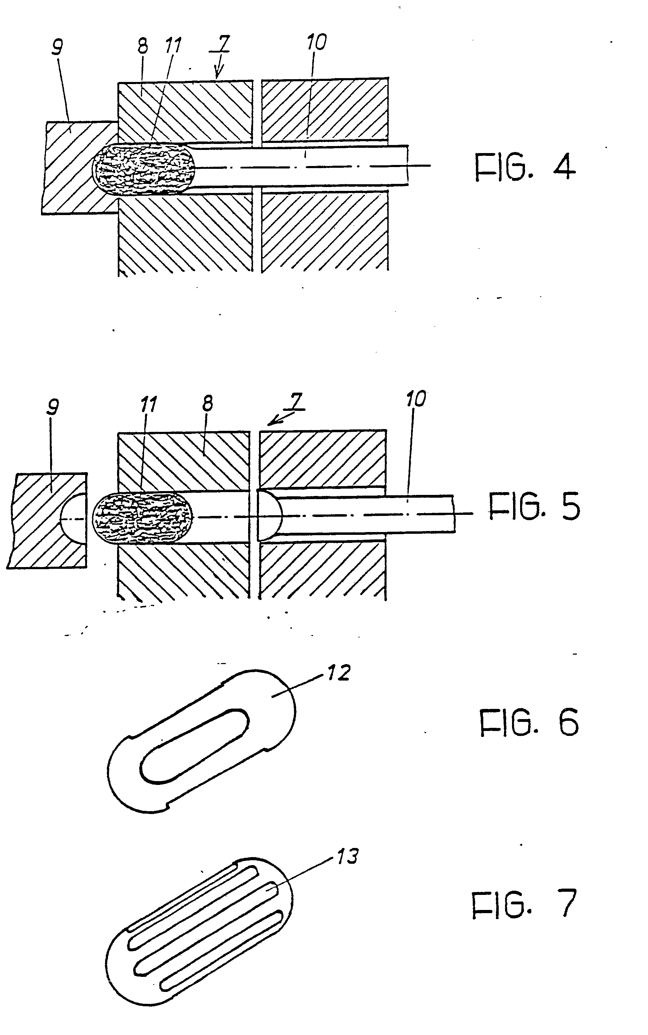

Aus der Backenpresse 2 wird der Faserrohling 6, z. B. mit Hilfe einer Schubstange, in eine geheizte, insgesamt mit 7 bezeichnete Preßtrommel nach Fig. 4 und 5 eingeschoben und axial komprimiert. In der Preßtrommel 7 paßt sich der Faserrohling in seiner Außenform den Konturen der Preßhülse 8, des kopfseitigen Verschlusses 9 und des Preßstößels 10 an. Eine saubere beständige Form des so hergestellten Duftträgers 11 wird nur erreicht, wenn alle Oberflächen der Preßtrommel 7 — also auch der kopfseitige Verschluß 9 und der Preßstößel 10 — beheizt werden. Nach dem Zurückfahren von Kopfverschluß 9 und Preßstößel 10 soll der fertige Duftträger 11 noch für eine gewisse Zeitdauer, z. B. 8-15 Sekunden lang, in der geheizten Formhülse 8 bleiben.

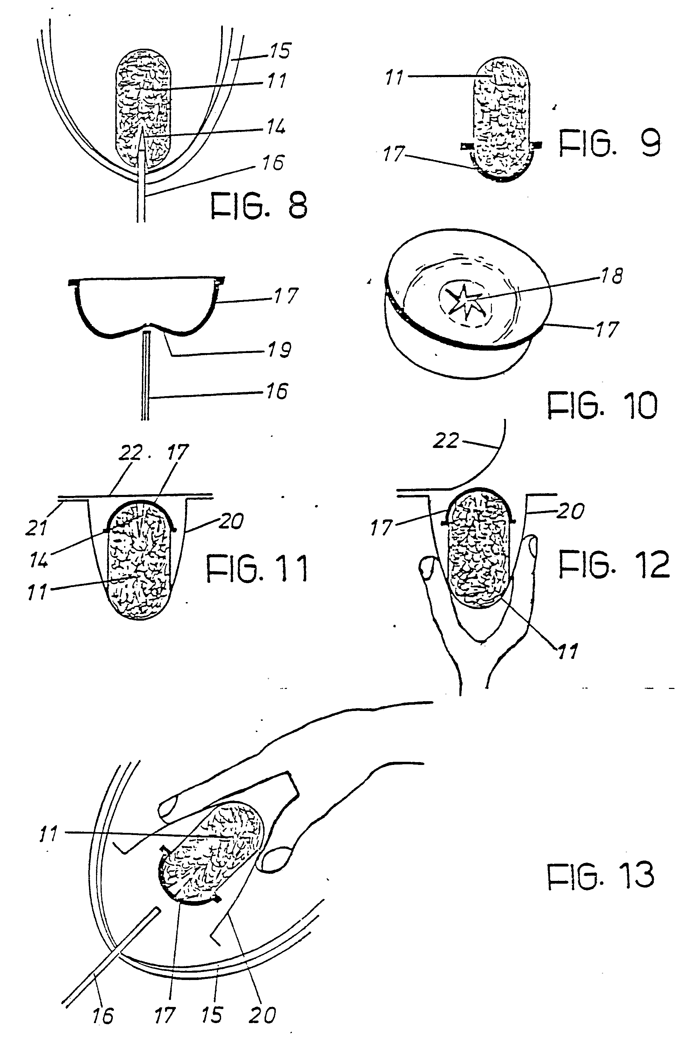

Bei der Formgebung in der Preßtrommel 7 kann der Duftträger 11 eine einem natürlichen Blütenstempel ähnliche Kontur gemäß Fig. 6 erhalten. Es kann außerdem günstig sein, durch Vorgabe der Form der Preßtrommel 7 in die abdampfende Oberfläche des Duftträgers 11 Rillen 13 nach Fig. 7 einzupressen. Schließlich kann der Duftträger 11 nach Fig. 8 oder 9 mit einer Einformung oder Anformung zu befestigen in der jeweiligen Kunststoffblume versehen werden. In Fig. 8 wird beispielsweise ein Duftträger 11 mit am unteren Ende vorgesehenen spitz zulaufenden Loch 14 dargestellt, in das ein im Blütenkelch 15 vorgesehener Draht 16 als Befestigung des Duftträgers einzusetzen ist.

Da naturgemäß der als Faserpreßling vorliegende Duftträger 11 keine große Maßgenauigkeit besitzt, kann es für die maschinelle Handhabung günstig sein, den Duftträger in einen Kunststoffnapf 17 nach Fig. 9 einzusetzen. Der Napfdurchmesser soll so bemessen werden, daß der Duftträger 11 klemmend im Napf 17 gehalten wird. Im Napfboden können sich nach Fig. 10 Klemmlippen 18 befinden, die gegebenenfalls den in der Kunstblume vorhandenen Draht 16 mit definierter Haltekraft umschließen sollen. Schließlich kann es nach Fig. 10 günstig sein, einen Einführtrichter 19 im Boden des Napfes 17 vorzusehen, damit das Aufstecken des Duftträgers auf den jeweiligen Haltedraht 16 der Kunstblume vereinfacht wird.

Da der Duftträger naturgemäß so aufgebaut wird, daß er Duftstoff abgibt, muß er bis zu seiner eigentlichen Anwendung möglichst diffusionsdicht verpackt werden. Gemäß weiterer Erfindung wird die Verpackung nicht nur als Diffusionsschutz sondern auch zum Beaufschlagen des Duftträgers mit dem Duftstoff und zugleich als Einführhilfe des Duftträgers in eine Kunstblume ausgebildet.

Eine diffusionsdichte Verpackung, die zudem aus wasser- und ölf/estem Material bestehen muß, läßt sich durch Verschweißen herstellen. Eine Benetzung der zu verschweissenden Flächen mit dem Duftstoff muß verhindert werden. Vorzugsweise wird als Verpackung ein tiefgezogener Becher aus Acrylnitril-Methylmetacrylat-Copolymer-Folie — kurz AMMA-Folie — verwendet.

In der Praxis kann der flüssige Duftstoff bereits in der Tiefziehmaschine in den Becher 20 eindosiert werden. Auf diese Weise wird ein Benetzen der zu verschweissenden Flächen am Becherrand 21 verhindert. Im nächsten Schritt wird der Duftträger 11 — mit seiner Kopfseite voran — in den Becher 20 eingelegt. Der Duftträger saugt den Duftstoff sofort auf. Unmittelbar nach dem Einlegen des Duftträgers 11 wird der Becher 20 mit einer diffusionsdichten Folie, vorzugsweise einer Aluminium-Verbundfolie, versiegelt. Gegebenenfalls erfolgt das Ausstanzen der verschlossenen Becher aus einem Folienband in üblicher Weise.

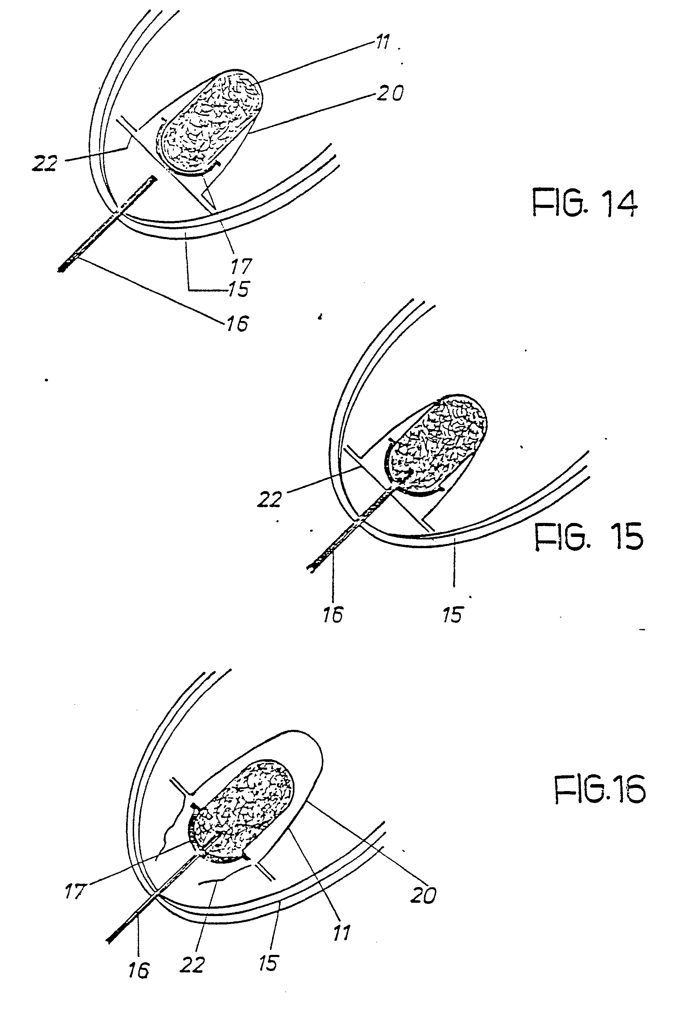

Beim Einsetzen des Duftträgers 11 in den Blütenkelch 15 einer Kunstblume wird der Becher 20 als Fingerschutz verwendet. Diese becherförmige Verpackung kann entweder als Peel-off-Packung nach Fig. 12 und 13 oder als Durchdrückpackung nach Fig. 14 bis 16 ausgebildet bzw. herangezogen werden.

Bei dem Handhaben als Peel-off-Packung nach Fig. 12 und 13 wird zunächst die Abdeckfolie 22 abgezogen und dann der Duftträger 11 — mit den Fingern im Becher 20 geklemmt — in den Blütenkelch 15 eingeführt und auf die im Ausführungsbeispiel als Draht 16 ausgebildete Klemmvorrichtung aufgesteckt. Anschließend kann die becherförmige Verpackung wieder aus dem Blütenkelch 15 herausgezogen werden. Beim Handhaben als Durchdrückpackung wird die Abdeckfolie 22 nach Fig. 14 und 15 mit dem Haltedraht 16 der Kunstblume durchstoßen. Der Duftträger 11 wird dann so weit in den Blütenkelch 15 eingeschoben, daß der Klemm-Mechanismus, beispielsweise die Klemmung des Drahtes 16 im Loch 14 (vgl. Fig. 8), faßt. Daraufhin wird die becherförmige Verpackung zurückgezogen. Dabei reißt die Folie 22 auf, so daß der Duftträger 11 gegebenenfalls gehalten in dem Kunststoffnapf 17 nach Fig. 16 zurückbleibt.

## Patentansprüche

1. Verfahren zum Herstellen des Duftträgers einer duftenden Kunstblume bestehend aus einem saugfähigen Material, wie Vliesstoff, mit den Duftstoff abdampfender Oberfläche, dadurch gekennzeichnet, daß ein aus Zellwollfasern bestehender Vliesstoff durch Nadeln mechanisch verfestigt wird und daß der verfestigte Faserrohling (6) in einer an allen Formflächen (8, 9, 10) beheizten Presse (7) komprimiert wird.

2. Verfahren nach Anspruch 1, dadurch gekennzeichnet, daß entsprechend der vorgesehenen Blumenfarbe mit öl- und wasserfester Farbe eingefärbte Fasern eingesetzt werden.

3. Verfahren nach Anspruch 1, dadurch gekennzeichnet, daß entsprechend der vorgesehenen Blumenfarbe spinndüsengefärbte Fasern eingesetzt werden.

4. Verfahren nach einem der Ansprüche 1 bis 3, gekennzeichnet durch folgende Reihe von Verfahrensschritten :

   a) Zellwollfasern werden in einem Öffnungsaggregat geöffnet und dann auf einer pneumatisch ablegenden Wirrvlieskarde zu einem Vlies gelegt ;

   b) das Vlies wird auf einer Nadelmaschine zu einem Vliesstoff mechanisch verfestigt ;

   c) der Vliesstoff wird geschnitten und stückweise auf einer Backenpresse (2) zu je einem radial verpreßten runden Faserrohling (6) verarbeitet ;

   d) der Faserrohling (6) wird, insbesondere mit Hilfe einer Schubstange, in eine allseits beheizte Preßtrommel (7) eingeschoben und dort axial verpreßt ;

   e) der so zu einem Duftträger (11) verpreßte Faserkörper wird nach dem Zurückfahren des Preßstempels (9, 10) vor der Entnahme für eine Fixierungszeit in der Preßform (7) belassen.

5. Verfahren nach einem der Ansprüche 1 bis 4, dadurch gekennzeichnet, daß als Ausgangsmaterial Zellwollfasern mit einem Titer von etwa 3,6 dtex und einer Stapellänge von etwa 30 mm verwendet werden.

6. Verfahren nach Anspruch 4 oder 5, dadurch gekennzeichnet, daß auf der Wirrvlieskarde ein Vlies mit etwa 600 g/m² Flächengewicht gelegt wird.

7. Verfahren nach einem der Ansprüche 4 bis 6, dadurch gekennzeichnet, daß der Vliesstoff vor dem Zuführen zu der Backenpresse (2) in Stückgrößen von etwa 30 × 40 mm geschnitten wird.

8. Verfahren nach einem der Ansprüche 4 bis 7, dadurch gekennzeichnet, daß der fertig verpreßte Duftträger (11) noch für eine Fixierzeit von etwa 8 bis 15 sec in der Preßform (7) belassen wird.

9. Verfahren nach einem der Ansprüche 4 bis 8, dadurch gekennzeichnet, daß die Backenpresse (2) zum Reduzieren der Faser-Metall-Reibung beheizt wird.

10. Verfahren nach einem der Ansprüche 1 bis 9, dadurch gekennzeichnet, daß der als Duftträger (11) vorgesehene Faserkörper mit einer Einformung (14), insbesondere als Loch zum Einstecken eines aus der jeweiligen Blume (15) vorstehenden Drahtes (16), ausgestattet wird.

11. Verfahren nach einem der Ansprüche 1 bis 10, dadurch gekennzeichnet, daß der als Dufträger (11) zu verwendende Faserkörper mit der Rückseite in einem vorzugsweise elastischen und ölfesten Kunststoffnapf (17) als Anpaßmittel zum Befestigen in einer Kunstblume (15) eingesetzt wird.

12. Verfahren nach Anspruch 1 oder 4, dadurch gekennzeichnet, daß der als Duftträger (11) vorgesehene Faserkörper mit der Kopfseite voran in einen flüssigen Duftstoff enthaltenden, aus diffusionsdichtem Material bestehenden Becher (20) eingelegt wird und daß der Becher (20) dann diffusionsdicht, insbesondere mit einer Folie (22), verschlossen wird.

13. Duftträger einer duftenden Kunstblume bestehend aus einem saugfähigen Material, wie Vliesstoff, mit den Dufststoff abdampfender Oberfläche, hergestellt nach dem Verfahren nach einem der Ansprüche 1 bis 12, gekennzeichnet durch einen Zellwollfaserkörper (11) mit einer Dichte von weniger als etwa 0,6 g/cm$^3$ und mindestens 0,4 g/cm$^3$ Duftstoffaufnahmekapazität.

14. Duftträger nach Anspruch 13, gekennzeichnet durch eine dem Blütenstempel der jeweiligen Kunstblume ähnliche Form.

15. Duftträger nach Anspruch 13 oder 14, dadurch gekennzeichnet, daß die abdampfende Oberfläche durch Rillung vergrößert ist.

16. Duftträger nach einem der Ansprüche 13 bis 15, gekennzeichnet durch eine Ein- oder Anformung (14, 17) zum Befestigen in der jeweiligen Kunstblume.

17. Duftträger nach Anspruch 16, gekennzeichnet durch ein spitz zulaufendes Loch (14) im unteren Ende zum Einsetzen eines aus dem Blütenkelch (15) der jeweiligen Kunstblume vorspringenden Drahtendes (16).

18. Duftträger nach Anspruch 16, gekennzeichnet durch einen klemmend auf die Rückseite aufgesetzten Napf (17), vorzugsweise aus elastischem und ölfestem Kunststoff.

19. Duftträger nach Anspruch 18, dadurch gekennzeichnet, daß der Napfboden Klemmlippen (18) zum definierten Festhalten eines aus der Kunstblume vorspringenden Drahtes besitzt.

20. Duftträger nach Anspruch 18 oder 19, gekennzeichnet durch einen Einführtrichter (19) zur Klemmvorrichtung am Napf.

21. Duftträger nach einem der Ansprüche 13 bis 20, gekennzeichnet durch eine den Duftstoff enthaltende und für den Duftstoff im wesentlichen diffusionsdichte Verpackung.

22. Duftträger nach Anspruch 21, dadurch gekennzeichnet, daß die Verpackung die Form eines Bechers (20) mit mit Hilfe einer Folie (22), z. B. Aluminiumverbundfolie, versiegelter Becheröffnung besitzt.

23. Duftträger nach Anspruch 22, dadurch gekennzeichnet, daß der Becher (20) aus tiefgezogener Akrylnitril-Methylmetacrylat-Copolymer-Folie besteht.

24. Duftträger nach Anspruch 22, gekennzeichnet durch die Ausbildung der Verpackung als Peel-off-Verpackung mit vor dem Einsetzen in die jeweilige Blume abzuziehender Folie (22).

25. Duftträger nach Anspruch 22, gekennzeichnet durch die Ausbildung der Verpackung als Durchdrückpackung mit mit Hilfe eines in der Kunstblume (15) vorgesehenen Haltedrahtes (16) zu durchstoßender Folie (22).

## Claims

1. Method for the manufacture of the scent carrier of a fragrant artificial flower consisting of an absorbent material, such as non-woven textile, with a surface evaporating the scent substance, characterised thereby, that a non-woven textile consisting of spun rayon fibres is strengthened mechanically by pinning and that the strengthened fibre blank (6) is compressed in a press (7) heated at all moulding surfaces (8, 9, 10).

2. Method according to claim 1, characterised thereby, that fibres are used, which have been coloured by oilproof and waterproof dye corresponding to the envisaged flower colour.

3. Method according to claim 1, characterised thereby, that fibres are used, which have been coloured in a spinning nozzle in correspondence with the envisaged flower colour.

4. Method according to one of the claims 1 to 3, characterised by the following series of method steps :

a) spun rayon fibres are opened up in an opening unit and then laid into a fleece on a pneumatically depositing random fleece teasel,

b) the fleece is strengthened mechanically into a non-woven textile on a pinning machine,

c) the non-woven textile is cut and processed piece by piece each into a radially compressed round fibre blank (6) on a jaw press (2),

d) the fibre blank (6) is pushed, in particular with the aid of a pushrod, into a press drum (7) heated at all sides and there compressed axially and

e) the fibre body thus compressed into a scent carrier (11) is left, after the return of the press die (9, 10) and before the removal, in the press mould (7) for a fixation time.

5. Method according to one of the claims 1 to 4, characterised thereby, that spun rayon fibres with a denier of about 3.6 dtex and a staple length of about 30 millimetres are used as starting material.

6. Method according to claim 4 or 5, characterised thereby, that a fleece with an areal weight of about 600 grams per square metre is laid on the random fleece teasel.

7. Method according to one of the claims 4 to 6, characterised thereby, that the non-woven textile is cut into piece sizes of about 30 by 40 millimetres before the feeding to the jaw press (2).

8. Method according to one of the claims 4 to 7, characterised thereby, that the finally compressed scent carrier (11) is still left in the press mould (7) for a fixation time of about 8 to 15 seconds.

9. Method according to one of the claims 4 to 8, characterised thereby, that the jaw press (2) is heated to reduce the fibremetal friction.

10. Method according to one of the claims 1 to 9, characterised thereby, that the fibre body provided as scent carrier (11) is equipped with an indentation (14), in particular as hole for the plugging-in of a wire (16) projecting out of the respective flower (15).

11. Method according to one of the claims 1 to 10, characterised thereby, that the fibre body to be used as scent carrier (11) is inserted by the rear side into a preferably elastic and oilproof synthetic material cup (17) as adapter for the fastening in an artificial flower (15).

12. Method according to claim 1 or 4, characterised thereby, that the fibre body provided as scent carrier (11) is laid with the head side forward into a beaker (20), which consists of diffusion-tight material and contains a liquid scent substance, and that the beaker (20) is then closed off in diffusion-tight manner, in particular by a film (22).

13. Scent carrier of a fragrant artificial flower consisting of an absorbent material, such as non-woven textile, with a surface evaporating the scent substance and manufactured by the method according to one of the claims 1 to 12, characterised by a spun rayon fibre body (11) with a density of less than about 0.6 grams per cubic centimetre and a scent substance take-up capacity of at least 0.4 grams per cubic centimetre.

14. Scent carrier according to claim 13, characterised by a shape similar to the pistil of the respective artificial flower.

15. Scent carrier according to claim 13 or 14, characterised thereby, that the evaporating surface is increased by grooving.

16. Scent carrier according to one of the claims 13 to 15, characterised by an indentation or protrusion (14, 17) for the fastening in the respective artificial flower.

17. Scent carrier according to claim 16, characterised by a hole (14), which converges to a point, at the lower end for the insertion of a wire end (16) projecting out of the calyx (15) of the respective artificial flower.

18. Scent carrier according to claim 16, characterised by a cup (17), which is wedgingly placed on the reaer side and preferably of elastic and oilproof synthetic material.

19. Scent carrier according to claim 18, characterised thereby, that the bottom of the cup possesses wedging lips (18) for the defined retention of a wire projecting from the artificial flower.

20. Scent carrier according to claim 18 or 19, characterised by an entry funnel (19) to the wedging device at the cup.

21. Scent carrier according to one of the claims 13 to 20, characterised by a packaging which contains the scent substance and is, substantially diffusion-tight for the scent substance.

22. Scent carrier according to claim 21, characterised thereby, that the packaging possesses the form of a beaker (20) with a beaker opening sealed with the aid of a film (22), for example compound aluminium film.

23. Scent carrier according to claim 22, characterised thereby, that the beaker (20) consists of deep-drawn acrylnitril methyl-metacrylate copolymer film.

24. Scent carrier according to claim 22, characterised by the construction of the packaging as peel-off packaging with a foil (22) to be pulled off before the insertion into the respective flower.

25. Scent carrier according to claim 22, characterised by the construction of the packaging as press-through packaging with a foil (22) to be pierced with the aid of a retaining wire (16) provided in the artificial flower (15).

**Revendications**

1. Procédé pour la fabrication d'un support de parfum d'une fleur artificielle parfumée, constitué d'une matière absorbante comme toison comportant une surface laissant s'évaporer le parfum, caractérisé par le fait que l'on consolide mécaniquement par aiguilletage une toison constituée de fibres de laine cellulosique et que l'on comprime l'ébauche en fibres consolidée (6) dans une presse (7) chauffée sur toutes les surfaces de moulage (8, 9, 10).

2. Procédé selon la revendication 1, caractérisé par le fait que conformément à la couleur de la fleur artificielle prévue, on utilise des fibres colorées par de la couleur résistant à l'huile et à l'eau.

3. Procédé selon la revendication 1, caractérisé par le fait que conformément à la couleur de la fleur artificielle prévue, on utilise des fibres colorées dans la filière.

4. Procédé selon l'une quelconque des revendications 1 à 3, caractérisé par le fait qu'il comporte la série d'étapes suivantes :

a) on ouvre des fibres de laine cellulosique dans un groupe d'ouverture et alors on les dépose en une toison sur une carde à toison emmêlée déposant pneumatiquement ;

b) on consolide mécaniquement la toison en une nappe sur une machine à aiguilleter ;

c) on coupe la nappe en pièces et, sur une presse à mâchoires (2), chaque pièce est transformée en une ébauche ronde en fibres (6) comprimées radialement ;

d) on enfonce chaque ébauche en fibres (6), en particulier à l'aide d'une tige de poussée, dans un tambour de compression (7) chauffé de tous côtés et on l'y comprime axialement ;

e) après rétraction du poinçon de presse (9, 10), on laisse dans le moule de compression (7), pendant un temps de fixage, le corps en fibres comprimé en un support de parfum, avant de le retirer.

5. Procédé selon l'une quelconque des revendications 1 à 4, caractérisé par le fait que comme matière première, on utilise des fibres de laine cellulosique ayant un titre d'environ 3,6 dtex et une longueur de coupe d'environ 30 mm.

6. Procédé selon l'une ou l'autre des revendications 4 ou 5, caractérisé par le fait que sur la carde à toison emmêlée, on dépose une toison ayant une masse surfacique d'environ 600 g/m².

7. Procédé selon l'une quelconque des revendications 4, 5, ou 6, caractérisé par le fait qu'avant de l'amener à la presse à mâchoires (2), on coupe la nappe en des pièces ayant environ 30 × 40 mm.

8. Procédé selon l'une quelconque des revendications 4 à 7, caractérisé par le fait qu'on laisse encore le support de parfum complètement comprimé (11) dans le moule de compression (7) pendant un temps de fixage d'environ 8 à 15 secondes.

9. Procédé selon l'une quelconque des revendications 4 à 8, caractérisé par le fait que l'on chauffe la presse à mâchoires (2) pour réduire le frottement fibres/métal.

10. Procédé selon l'une quelconque des revendications 1 à 9, caractérisé par le fait que l'on fait dans le corps en fibres, prévu comme support de parfum (11), une forme de pénétration (14) en particulier sous forme d'un trou pour l'insertion d'un fil (16) qui dépasse de la fleur artificielle (15) dont il s'agit.

11. Procédé selon l'une quelconque des revendications 1 à 10, caractérisé par le fait que l'on insère le corps en fibres, à utiliser comme support de parfum (11), par le côté postérieur, dans un godet en matière synthétique (17) de préférence élastique et résistant à l'huile, comme moyen d'adaptation pour la fixation dans une fleur artificielle (15).

12. Procédé selon l'une ou l'autre des revendications 1 ou 4, caractérisé par le fait que l'on insère le corps en fibres, à utiliser comme support de parfum (11), le côté de tête en avant, dans un godet (20) constitué de matière étanche à la diffusion, et qu'alors, on ferme ce godet (20) de façon étanche à la diffusion, en particulier au moyen d'une feuille (22).

13. Support de parfum de fleur artificielle parfumée, constitué d'une matière absorbante comme une toison comportant une surface laissant s'évaporer le parfum, fabriqué selon le procédé selon l'une quelconque des revendications 1 à 12, caractérisé par le fait qu'il est constitué d'un corps en fibres de laine cellulosique (11) ayant une masse volumique inférieure à environ 0,6 g/cm³ et une capacité d'absorption de parfum d'au moins 0,4 g/cm³.

14. Support de parfum selon la revendication 13 caractérisé par le fait qu'il a une forme analogue au pistil de la fleur artificielle dont il s'agit.

15. Support de parfum selon l'une ou l'autre des revendications 13 ou 14, caractérisé par le fait que la surface d'évaporation est agrandie par rainurage.

16. Support de parfum selon l'une quelconque des revendications 13, 14 ou 15, caractérisé par le fait qu'il comporte une forme de pénétration (14, 17) pour la fixation dans la fleur artificielle dont il s'agit.

17. Support de parfum selon la revendication 16, caractérisé par le fait qu'il comporte un trou borgne (14), à fond conique, pour l'insertion d'une extrémité de fil (16) dépassant du calice (15) de la fleur artificielle dont il s'agit.

18. Support de parfum selon la revendication 16, caractérisé par le fait qu'il comporte un godet (17), de préférence en matière synthétique et résistant à l'huile, posé avec serrage sur le côté postérieur.

19. Support de parfum selon la revendication 18, caractérisé par le fait que le fond du godet présente des lèvres de serrage (18) pour la retenue définie d'un fil dépassant de la fleur artificielle.

20. Support de parfum selon l'une ou l'autre des revendications 18 ou 19, caractérisé par le fait qu'il comporte un entonnoir d'introduction (19) pour le dispositif de serrage sur le godet.

21. Support de parfum selon l'une quelconque des revendications 13 à 20, caractérisé par le fait qu'il est pourvu d'un emballage contenant le parfum et pratiquement étanche à la diffusion du parfum.

22. Support de parfum selon la revendication 21, caractérisé par le fait que l'emballage présente la forme d'un godet (20) avec ouverture de godet scellée à l'aide d'une feuille (22), par exemple d'une feuille composite d'aluminium.

23. Support de parfum selon la revendication 22, caractérisé par le fait que le godet (20) est constitué d'une feuille emboutie de copolymère acrylonitrile/méthacrylate de méthyle.

24. Support de parfum selon la revendication 22, caractérisé par le fait que l'emballage est constitué sous forme d'un emballage avec enveloppe déchirable et feuille (22) à retirer avant l'insertion dans la fleur artificielle dont il s'agit.

25. Support de parfum selon la revendication 22, caractérisé par le fait que l'emballage est constitué sous forme d'un emballage à enfoncement, avec feuille (22) à percer à l'aide d'un fil de retenue (16) prévu dans la fleur artificielle (15) dont il s'agit.

0 163 891

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

3

FIG. 14

FIG. 15

FIG.16